Europäisches Patentamt

European Patent Office

Office européen des brevets

(11) Numéro de publication: **0 386 620 B1**

# FASCICULE DE BREVET EUROPEEN

(45) Date de publication de fascicule du brevet: **25.05.94**  (51) Int. Cl.[5]: **A61B 5/02**, G06F 15/20

(21) Numéro de dépôt: **90103965.1**

(22) Date de dépôt: **01.03.90**

(54) **Procédé et dispositif pour établir la relation pression-diamètre d'une artère par des mesures non invasives.**

(30) Priorité: **08.03.89 CH 855/89**
  **13.03.89 FR 8903364**

(43) Date de publication de la demande:
  **12.09.90 Bulletin 90/37**

(45) Mention de la délivrance du brevet:
  **25.05.94 Bulletin 94/21**

(84) Etats contractants désignés:
  **DE DK ES GB IT NL SE**

(56) Documents cités:
  **EP-A- 0 075 284**
  **FR-A- 2 481 917**

  **MEDICAL & BIOLOGICAL ENG. & COMP., Vol. 16, No. 6, novembre 1978, pages 715-726, London GB, D.N. GHISTA et al.: "Analysis for the non-invasive determination of arterial properties and for the transcutaneous continuous monitoring of arterial blood pressure"**

  **IEEE TRANSACTION ON BIOMEDICAL ENG., Vol. BME-27, No. 3, mars 1980, pages 150-155, New York US; K. YAMAKOSHI et al.: "Indirect Measurement of instantaneous Arterial Blood Pressure in the Human Finger**

  **by the Vascular Un loading Technique"**

(73) Titulaire: **ASULAB S.A.**
  **Faubourg du Lac 6**
  **CH-2501 Bienne(CH)**

(72) Inventeur: **Meister, Jean-Jacques**
  **Chemin de l'Eglise 10**
  **CH-1066 Epalinges(CH)**
  Inventeur: **Tardy, Yanik**
  **Rue du Crêt 4bis**
  **CH-1006 Lausanne(CH)**

(74) Mandataire: **de Raemy, Jacques et al**
  **ICB**
  **Ingénieurs Conseils en Brevets SA**
  **Passage Max. Meuron 6**
  **CH-2001 Neuchâtel (CH)**

## Description

L'invention est relative à un procédé pour établir la relation pression-diamètre d'une artère en un point donné de son parcours et à un dispositif pour la mise en oeuvre de ce procédé.

On sait que la compliance artérielle, c'est-à-dire le comportement élastique de l'artère, est considérée comme indispensable à la bonne connaissance de la physiologie, la physiopathologie et la thérapeutique du système artériel. Cette compliance est fonction de la pression artérielle et l'on a donc besoin, pour l'établir, de la relation instantanée qui existe entre la pression et le diamètre en un point donné de l'artère.

Des propositions pour mesurer la relation pression-diamètre ont déjà été faites, par exemple dans l'étude présentée aux pages 789 à 793 de la revue Arch. Mal. Coeur, no 6, 1987, où le comportement visco-élastique de l'aorte chez le chien conscient est analysé. La réponse visco-élastique de l'aorte à l'administration d'hormones est observée dans l'étude citée en analysant la relation pression-diamètre aortique. Cette relation est établie au moyen d'un microcapteur de pression, calibrable in situ introduit à travers l'artère humérale gauche et placé dans la lumière de l'aorte descendante et de deux cristaux piézoélectriques de 4 mm de diamètre diamétralement fixés dans l'adventice de l'aorte descendante proximale.

Les moyens qui viennent d'être évoqués ont un caractère invasif, c'est-à-dire touchent à l'intégrité des organes sur lesquels ils interviennent. Au contraire de cela, le procédé et le dispositif pour sa mise en oeuvre, objets de la présente invention, font appel à des capteurs non invasifs, restant disposés à la superficie de l'artère à analyser sans aucune pénétration dans les tissus environnants.

Des capteurs non invasifs permettant la mesure continue de la pression sanguine sont connus. Il s'agit notamment du photopléthysmographe commercialisé par la Société Ohmeda, 3030 Airco Drive, Madison, Wisconsin, USA et portant la marque déposée "finapres" (pour finger arterial pressure). Comme indiqué, l'appareil mesure la pression sanguine à l'extrémité d'un doigt selon la manière décrite dans l'article "Effects of peripheral vasoconstriction on the measurement of blood pressure in a finger" dans la revue Cardiovascular Research, 1985, 19, 139-145.

Des capteurs non invasifs permettant la mesure du diamètre artériel sont également connus. Il s'agit notamment de l'appareil utilisé dans le document US-A-4,370,985 qui permet la mesure du diamètre de l'artère par l'envoi d'une onde ultrasonique sur l'artère et la mesure des échos renvoyés par les parois de l'artère. Cette mesure de diamètre peut s'effectuer sur des artères superficielles, par exemple l'artère humérale ou l'artère radiale.

De la brève description des capteurs connus actuellement qui vient d'être donnée ci-dessus, il ressort qu'il n'est pas possible de mesurer non invasivement la pression dans toute artère autre que celle du doigt et le diamètre de ladite artère au même endroit de sorte que la relation ou courbe pression-diamètre présente une hystérèse systématique. Cela est dû au fait que, la vitesse de propagation de l'onde étant finie, les variations de pression mesurées en aval accusent un certain retard par rapport aux variations du diamètre correspondant. Ce retard est bien sûr plus important lorsque la distance qui sépare les deux sites de mesure augmente. Ce défaut de la mesure doit donc être corrigé pour que les propriétés mécaniques de l'artère, calculées à partir de la relation pression-diamètre, ne soient pas artificiellement faussées.

C'est le but de la présente invention de proposer un procédé pour rapporter des mesures de pression faites à un endroit du lit artériel à un autre endroit où est mesuré le diamètre de l'artère. Pour cela, le procédé est caractérisé en ce qu'il comporte la succession des étapes suivantes :

a) on mesure de façon non invasive et simultanément pendant au moins un cycle cardiaque, d'une part le diamètre $D(t)$ de l'artère en un premier endroit et d'autre part la pression $p(t)$ du lit artériel en un second endroit, lesdits premier et second endroits étant supposés séparés par une distance $\Delta x$.

b) on mémorise en des instants successifs du cycle cardiaque des couples de valeurs comportant une valeur de diamètre $D(t)$ et une valeur de pression $p(t)$,

c) on calcule par une méthode mathématique d'ajustement sur les couples de valeurs ainsi mémorisés les paramètres $\alpha, \beta, \gamma, ...$ d'une relation du diamètre en fonction de la pression $D(p) = D(p, \alpha, \beta, \gamma, ...)$, relation sélectionnée pour rendre compte du comportement de l'artère,

d) on calcule, à partir desdits paramètres et de chaque valeur de pression $p(t)$ initialement mesurée, la vitesse de propagation $c(p)$ de l'onde de pression engendrée par la fonction cardiaque,

e) on calcule pour chaque valeur de vitesse de propagation $c(p)$ ainsi établie et compte tenu de' ladite distance $\Delta x$ le temps de parcours $\Delta t(p) = \Delta x/c(p)$ de l'onde de pression entre lesdits premier et second endroits,

f) on calcule pour chaque valeur de la pression $p(t)$ initialement mesurée, une nouvelle valeur de pression $p[t + \Delta t(p)]$ régnant audit premier endroit et

g) on établit, à l'aide de la première valeur de diamètre $D(t)$ initialement mesurée au premier endroit et de ladite nouvelle valeur de pression, la courbe pression-diamètre D(p) de ladite artère audit premier endroit.

C'est aussi le but de la présente invention de proposer un dispositif pour mettre en oeuvre le procédé défini ci-dessus. Ce dispositif est caractérisé par le fait qu'il comporte deux capteurs non invasifs, le premier étant un capteur de diamètre placé en un premier endroit et le second étant un capteur de pression placé en un second endroit du parcours d'une artère, lesdits premier et second endroits étant supposés séparés par une distance Δx, et en ce qu'il comporte en outre un calculateur pour le traitement des valeurs livrées par lesdits capteurs et un écran de visualisation.

L'invention va être comprise maintenant à la lumière de la description suivante donnée à titre d'exemple et en s'aidant du dessin dans lequel :

- la figure 1 est une vue schématique du dispositif de mesure selon l'invention montrant un bras terminé par une main, des capteurs de diamètre D(t) et de pression p(t) étant disposés respectivement sur l'artère humérale de bras et sur un doigt de la main et un calculateur équipé d'un écran de visualisation,
- la figure 2 est un diagramme montrant le signal D(t) capté par le capteur de diamètre schématisé à la figure 1,
- la figure 3 est un diagramme montrant le signal p(t) capté par le capteur de pression schématisé à la figure 1,
- la figure 4 est un diagramme montrant un ensemble de points D(p) résultant de la combinaison des diagrammes des figures 2 et 3,
- la figure 5 est un diagramme montrant une courbe résultant d'un ajustement effectué sur l'ensemble de points du diagramme de la figure 4,
- la figure 6 est un diagramme montrant un ensemble de points D(p) pour lesquels la mesure de la pression p(t) a été reporté à l'endroit de la mesure du diamètre, cet ensemble présentant une correction qui n'est pas optimale,
- la figure 7 est un diagramme montrant un ensemble de points D(p) pour lesquels la mesure de la pression p(t) a été reporté à l'endroit de la mesure de diamètre, cet ensemble présentant une correction qui est optimale,
- la figure 8 reprend l'ensemble de points D(p) montré en figure 7, ensemble au moyen duquel on obtient la courbe pression-diamètre définitive,
- la figure 9 est un diagramme représentant la compliance de l'artère en fonction de la pression, diagramme résultant de la courbe obtenue en figure 8,
- la figure 10 est un diagramme représentant la vitesse de l'onde de pression en fonction de la pression, diagramme résultant de la courbe obtenue en figure 8 et
- la figure 11 est un organigramme montrant comment s'enchaînent les diverses étapes du procédé selon l'invention.

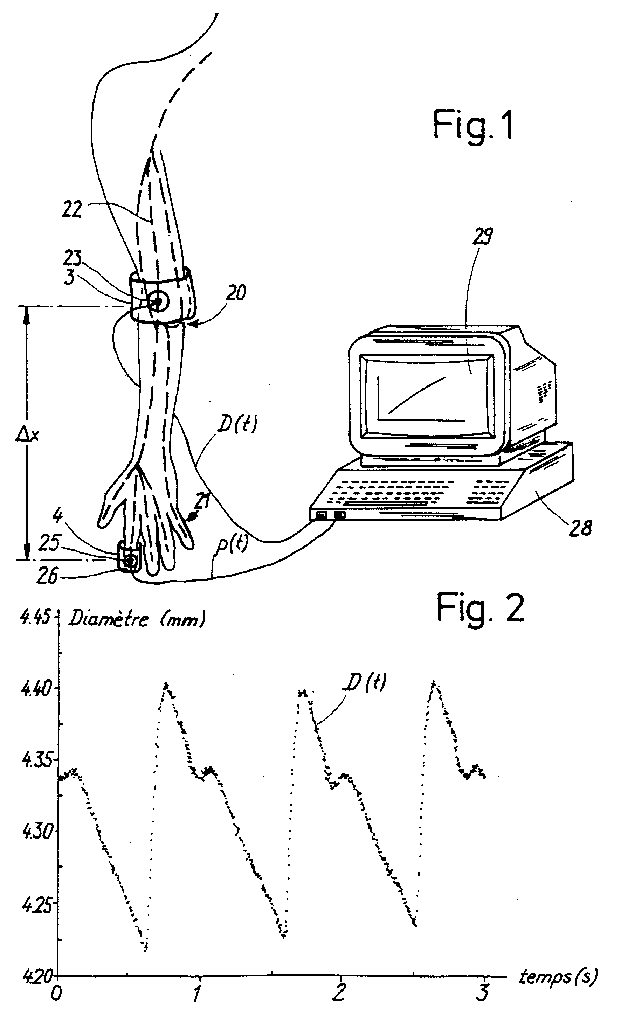

La figure 1 est une vue schématique d'un bras 20 terminé par une main 21. Dans ce bras on trouve une artère superficielle 22. Il s'agit de mesurer en un point donné du parcours de l'artère la courbe pression-diamètre. Pour ce faire, on dispose en un premier endroit 23, sur l'artère humérale, un capteur 3 permettant de mesurer le diamètre D(t) de ladite artère. Comme on l'a dit plus haut ce capteur non invasif peut être du type à émission ultrasonore captant les échos sur les parois de l'artère. On dispose également en un second endroit 25, à l'extrémité d'un doigt 26, un capteur 4 permettant de mesurer la pression p(t). Ce capteur est non invasif et est du type pléthysmographe comme évoqué plus haut. La mesure du diamètre et de la pression se fait simultanément pendant au moins un cycle cardiaque. Les endroits de mesure 23 et 25 sont séparés par une distance Δx. Les signaux D(t) et p(t) issus respectivement des capteurs 3 et 4 sont envoyés à un calculateur 28 pour traitement. Le calculateur est complété par un écran de visualisation 29.

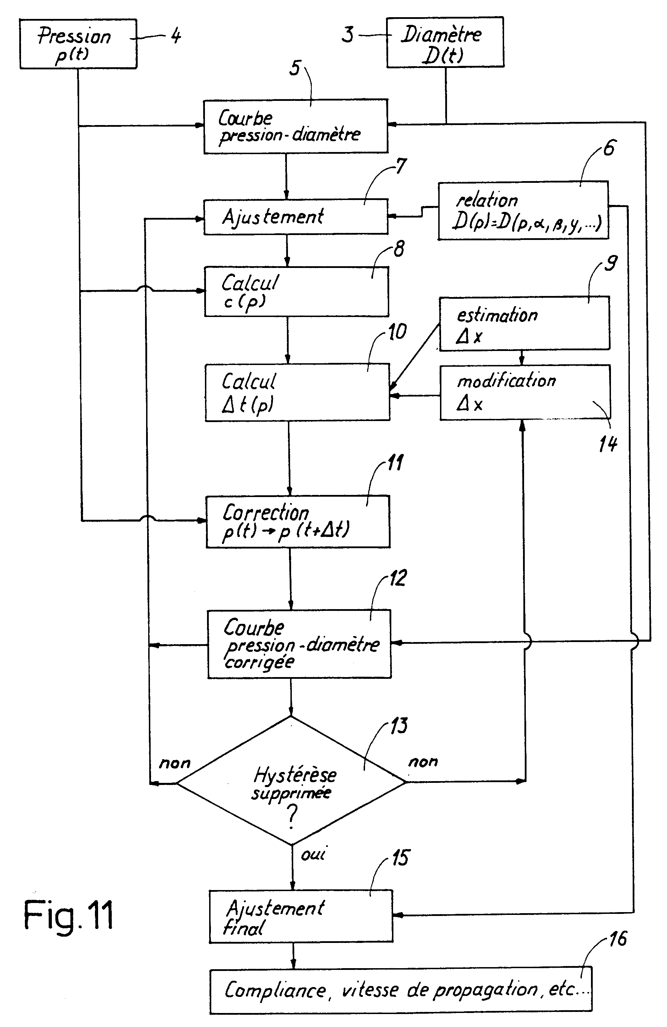

A l'aide de l'arrangement qui vient d'être décrit, le procédé pour établir la courbe pression-diamètre d'une artère en un point donné 23 de son parcours va être expliqué maintenant à l'aide de l'organigramme de la figure 11 et des divers diagrammes montrés aux figures 2 à 8.

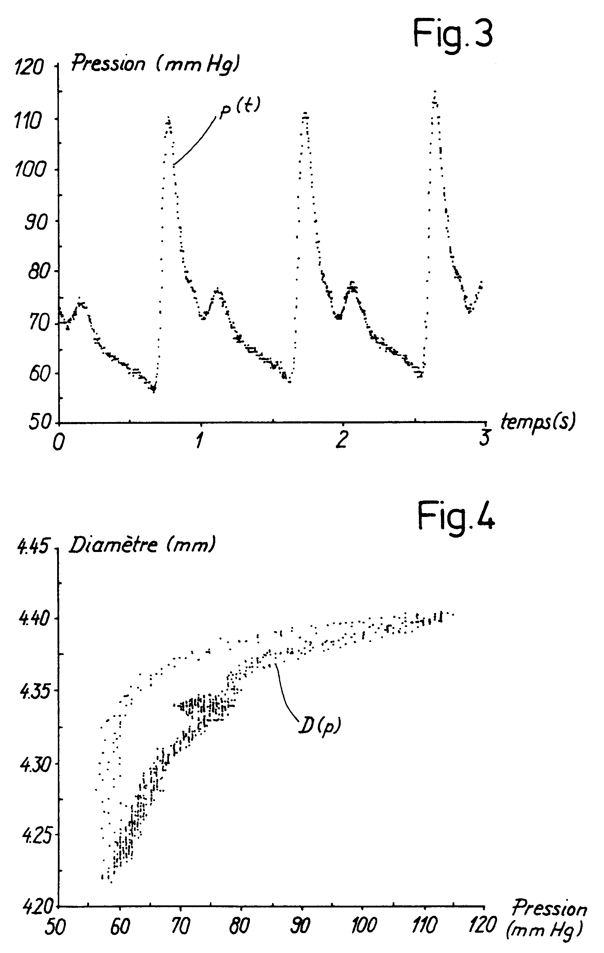

La figure 2 montre le signal D(t) issu du capteur de diamètre 3, où les valeurs de diamètre de l'artère sont représentées en fonction du temps sur environ trois cycles cardiaques. Simultanément à ces mesures de diamètre, le capteur de pression 4 délivre un signal p(t), illustré également sur environ trois cycles cardiaques à la figure 3, signal qui donne les valeurs de pression en fonction du temps. On rappelle ici, et cela pour les raisons qui ont été données plus haut, que la mesure de diamètre est effectuée en un premier endroit 23 (sur l'artère humérale 22) et que la mesure de pression est effectuée en un second endroit 25 (à l'extrémité du doigt 26), ces deux endroits étant séparés par la distance Δx. Ainsi pour une valeur de temps t1 on dispose d'un couple de valeurs D(t1) et p(t1) et ainsi de suite pour d'autres valeurs du temps.

On mémorise ensuite dans le calculateur 28, et en des instants t successifs du cycle cardiaque, les couples de valeurs ainsi mesurés. Puisqu'en chaque instant on dispose d'un couple de valeurs D et p, il est possible maintenant d'établir un diagramme où le diamètre D est fonction de la pression p, la variable temps ayant été éliminée. Ce diagramme est montré en figure 4, où apparaît un ensemble de points D(p) qui font apparaître une hystérèse marquée, due au fait de la distance $\Delta x$ qui existe entre les deux endroits de mesure, comme cela a été expliqué ci-dessus. Le diagramme pression-diamètre apparaissant en figure 4 est stocké dans le calculateur 28 dans son bloc 5 (figure 11). Sur demande, ce diagramme peut être visualisé sur l'écran 29.

On va calculer maintenant, par une méthode mathématique d'ajustement sur les couples de valeurs mémorisés précédemment les paramètres $\alpha,\beta,\gamma,...$ d'une relation du diamètre en fonction de la pression D(p) = D(p,$\alpha,\beta,\gamma$, ...), relation sélectionnée pour rendre compte du comportement de l'artère.

Ainsi, on choisit d'abord une relation D(p) relatif au comportement pression-diamètre de l'artère qu'on stocke dans le bloc 6 du calculateur 28. Cette relation est donnée par l'expérience. Elle pourrait être de la forme exponentielle :

$$p = \alpha e^{\dfrac{\beta \pi D^2}{4}}$$

où D est le diamètre de l'artère, ou encore d'une forme donnée dans la revue J. Biomechanics, Vol 17, no 6, pp. 425-435, 1984 et qui s'écrit :

$$S = \alpha \, [1/2 + \tan^{-1} [(p - \beta) / \gamma]/\pi]$$

où S est la section de l'artère. Dans les nombreuses relations proposées dans la littérature, le nombre de paramètres $\alpha,\beta,\gamma,...$ est variable.

Ayant choisi la relation D(p) = D(p,$\alpha,\beta,\gamma$, ...) convenable, on va procéder à son ajustement sur les couples de valeurs précédemment obtenus et ceci au moyen d'une méthode mathématique d'ajustement ou routine connue de l'état de l'art, par exemple la méthode des moindres carrés. D'autres méthodes sont possibles et sont décrites en détail dans l'ouvrage "Numerical Recipes" publié par "The Press Syndicate of the University of Cambridge" 1986. Très généralement, il s'agit d'un procédé mathématique standard de minimisation des écarts.

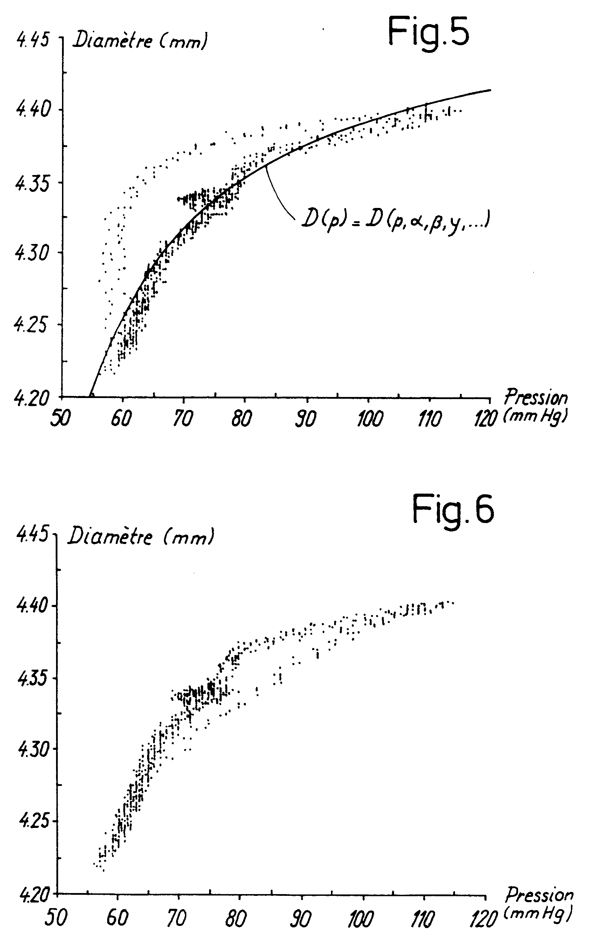

Cet ajustement (ou "fit"), qui est symbolisé par le bloc 7 de l'organigramme de la figure 11, donne naissance à la courbe pression-diamètre D(p) = D(p,$\alpha,\beta,\gamma$,...) dessinée sur la figure 5, courbe déterminée par les paramètres $\alpha,\beta,\gamma,...$

L'étape suivante (bloc 8 de l'organigramme de la figure 11) permet de calculer, à partir des paramètres $\alpha,\beta,\gamma,...$ précédemment obtenus et pour chaque valeur de pression p(t) initialement mesurée (bloc 4), la vitesse de propagation c(p) de l'onde de pression engendrée par la fonction cardiaque. La vitesse c(p) peut être obtenue par exemple en utilisant son expression connue par l'étude de l'hémodynamique artérielle et qui s'écrit

$$c(p) = \sqrt{\frac{S}{\rho} \cdot \frac{dp}{dS}}$$

où $\rho$ est la densité du sang,

$$S = \frac{\pi D^2}{4}$$

est la section de l'artère et où $\frac{dp}{dS}$ est la dérivée de la pression par la section.

On calcule ensuite pour chaque valeur de vitesse de propagation c(p) obtenue à l'étape précédente et, compte tenu de la distance $\Delta x$ séparant les premier et second endroits de mesure (23 et 25 de la figure 1), le temps

$$\Delta t(p) = \Delta x/c(p)$$

que met l'onde de pression à parcourir ladite distance $\Delta x$. Ce calcul est effectué par le bloc 10. On fera remarquer ici que la distance $\Delta x$ est donnée par estimation (bloc 9). Une valeur approximative de ce $\Delta x$ est obtenue en mesurant extérieurement la distance qui sépare les deux sites de mesure.

Pour chaque valeur de la pression p(t) initialement mesurée (bloc 4), on calcule ensuite une nouvelle valeur de la pression p[t + $\Delta t(p)$] régnant ou, en d'autres termes, rapportée au premier endroit et qui exprime la valeur de la pression qu'on aurait mesurée à ce premier endroit, si le capteur de pression avait pu s'y trouver. Cette correction est effectuée par le bloc 11 de l'organigramme de la figure 11.

Enfin, on établit, à l'aide de la valeur de diamètre D(t) initialement mesurée au premier endroit (bloc 3) et de la nouvelle valeur de pression p[t + $\Delta t(p)$] précédemment obtenue, la relation pression-diamètre D(p) au premier endroit (23). Cette courbe est présente au bloc 12.

Les étapes successives qui ont été expliquées ci-dessus donnent ainsi le procédé complet revendiqué de la présente invention selon la première revendication et qui permet de mesurer la relation pression-diamètre d'une artère en un point donné de son parcours, alors même qu'au moins une des mesures ne s'effectue pas en ce point.

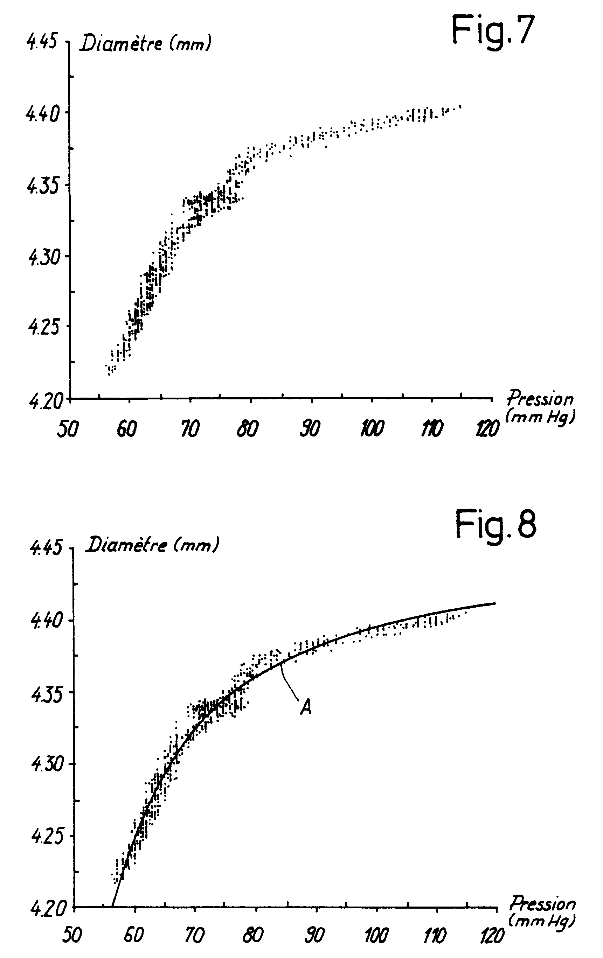

On remarquera ici que la courbe pression-diamètre obtenue au bloc 12 est un ensemble de points qui peuvent se présenter de différentes façons. La figure 7 montre un graphique ne présentant plus d'hystérèse. La correction est donc jugée optimale. Dans ces conditions le bloc 13, dit bloc de décision, fait procéder à un ajustement final (bloc 15) en utilisant à nouveau un procédé mathématique standard de minimisation des écarts, comme décrit plus haut. On tire alors de cet ajustement les valeurs définitives des paramètrès $\alpha$, $\beta$, $\gamma$,... caractéristiques de l'artère observée. L'ajustement final est montré par la courbe pleine A de la figure 8 qui se présente comme une moyenne superposée à l'ensemble de points de la figure 7 et repris en figure 8.

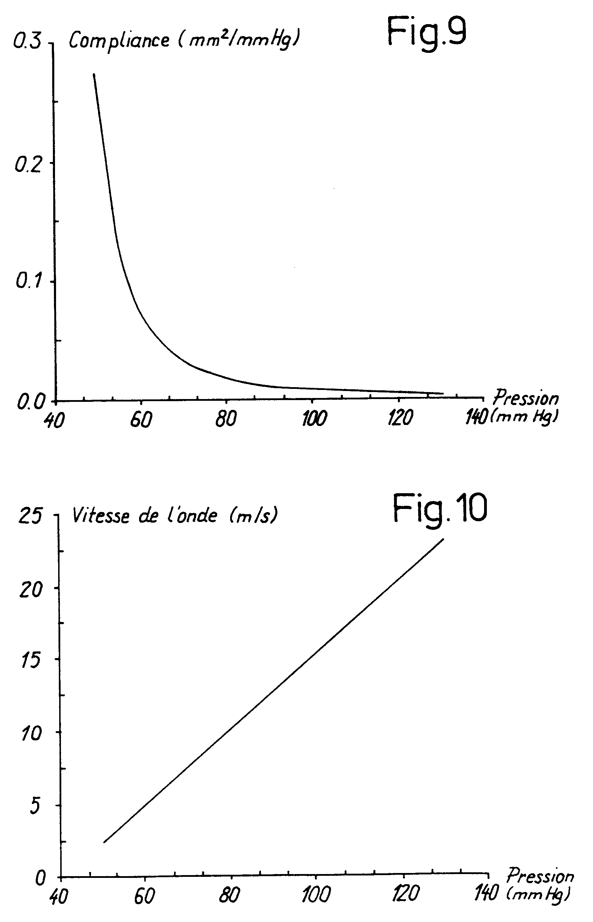

Le calcul des propriétés mécaniques de l'artère est alors effectuée à partir de la courbe A de la figure 8 (bloc 16). De cette courbe se déduit la compliance (figure 9) défini par le rapport dS/dp, qui n'est pas autre chose que la pente de la courbe A, et la vitesse de propagation de l'onde de pression c(p) (figure 10).

La courbe pression-diamètre obtenue au bloc 12 peut présenter encore une certaine hystérèse, commme c'est le cas dans l'ensemble de points montré en figure 6. Dans ce cas le bloc de décision 13 jugera la correction comme non optimale. Les valeurs obtenues au bloc 12 feront alors l'objet d'un nouvel ajustement en reprenant le procédé décrit à partir du bloc 7 et en parcourant à nouveau les étapes des blocs 8 à 12 et ceci aussi longtemps que la dispersion des couples de valeurs obtenus après l'étape symbolisée par le bloc 12 ne satisfait pas un critère prédéterminé qui est en fait la disparition de l'hystérèse. On procéde donc ici par itérations successives.

L'hystérèse peut encore être corrigée d'une autre façon, à savoir en modifiant la valeur de $\Delta x$ prise initialement (bloc 9). Si l'organe de décision 13 indique une hystérèse non supprimée, le calculateur peut modifier la valeur de $\Delta x$ (bloc 14) et l'introduire à partir de l'étape calculant le retard $\Delta x$ (bloc 10). A ce moment le calcul est repris à partir du bloc 10. Il est clair qu'on peut combiner la modification de $\Delta x$ et l'ajustement opéré à partir du bloc 7.

On mentionnera encore que toutes les étapes du procédé de l'invention, ainsi que les calculs qui apparaissent dans l'organigramme de la figure 11 peuvent être réalisés au moyen d'un ordinateur vendu sur le marché, par exemple au moyen de l'ordinateur de la marque Olivetti M 28. De même, comme cela a été dit plus haut à propos du bloc 5, l'écran de visualisation permet de faire apparaître, à la demande, n'importe quel graphique du procédé si le médecin traitant le juge nécessaire.

**Revendications**

1. Procédé pour établir la relation pression-diamètre d'une artère (22) en un point donné (23) de son parcours, caractérisé par le fait qu'il comporte la succession des étapes suivantes :
   a) on mesure de façon non invasive et simultanément pendant au moins un cycle cardiaque, d'une part le diamètre D(t) de l'artère en un premier endroit (23) et d'autre part la pression p(t) du lit artériel en un second endroit (25), lesdits premier et second endroits étant supposés séparés par une distance $\Delta x$,
   b) on mémorise (3, 4, 5) en des instants successifs du cycle cardiaque des couples de valeurs comportant une valeur de diamètre D(t) et une valeur de pression p(t),
   c) on calcule (7, 6) par une méthode mathématique d'ajustement sur les couples de valeurs ainsi mémorisés les paramètres $\alpha,\beta,\gamma$,... d'une relation du diamètre en fonction de la pression D(p) = D(p,$\alpha$,$\beta$,$\gamma$,...), relation sélectionnée pour rendre compte du comportement de l'artére,
   d) on calcule (8), à partir desdits paramètres et de chaque valeur de pression p(t) initialement mesurée, la vitesse de propagation c(p) de l'onde de pression engendrée par la fonction cardiaque,
   e) on calcule (10) pour chaque valeur de vitesse de propagation c(p) ainsi établie et compte tenu de ladite distance $\Delta x$ (9) le temps de parcours $\Delta t(p) = \Delta x/c(p)$ de l'onde de pression entre lesdits premier et second endroits,
   f) on calcule (11) pour chaque valeur de la pression p(t) initialement mesurée, une nouvelle valeur de pression p[t + $\Delta t(p)$] régnant audit premier endroit et
   g) on établit (12) à l'aide de la valeur de diamètre D(t) initialement mesurée et de ladite nouvelle valeur de pression au premier endroit, la relation pression-diamètre D(p) de ladite artère audit premier endroit.

2. Procédé selon la revendication 1, caractérisé par le fait que
   h) on répète (13) les étapes c) à g) jusqu'à ce que la dispersion des couples de valeurs obtenus après l'étape g), satisfasse un critère prédéterminé.

3. Procédé selon la revendication 2, caractérisé par le fait que l'étape h) est effectuée en reprenant les valeurs acquises à la suite de l'étape g).

**4.** Procédé selon la revendication 2, caractérisé par le fait que l'étape h) est effectuée en reprenant les valeurs acquises à la suite de l'étape g) et en modifiant la valeur de ladite distance Δx (14) supposée quand on procéde au calcul indiqué lors de l'étape e).

**5.** Procédé selon la revendication 1, caractérisé par le fait que

i) on répète les étapes e) à g) jusqu 'à ce que la dispersion des couples de valeurs obtenus après l'étape g) satisfasse un critère prédéterminé en modifiant (14) la valeur de ladite distance Δx supposée.

**6.** Procédé selon la revendication 1, caractérisé par le fait que, pour calculer la vitesse de propagation c(p) de l'étape d), on utilise l'expression

$$c(p) = \sqrt{\frac{S}{\rho} \cdot \frac{dp}{dS}}$$

où

$$S = \frac{\pi D^2}{4}$$

et où $\rho$ est la densité du sang.

**7.** Procédé selon la revendication 1, caractérisé par le fait qu'on visualise sur un écran (29) tout ou partie des résultats obtenus.

**8.** Dispositif pour la mise en oeuvre du procédé selon la revendication 1, caractérisé par le fait qu'il comporte deux capteurs non invasifs, le premier étant un capteur (3) de diamètre placé en un premier endroit (23) et le second étant un capteur (4) de pression placé en un second endroit (25) du parcours d'une artère (22), lesdits premier (23) et second (25) endroits étant supposés séparés par une distance Δx, et en ce qu'il comporte en outre un calculateur (28) pour le traitement des valeurs livrées par lesdits capteurs (3, 4) et un écran de visualisation (29).

**Claims**

**1.** Method for establishing the pressure-diameter relationship of an artery (22) at a given point (23) in its course, characterized by the fact that it includes the succession of the following steps :

a) measuring non-invasively and simultaneously during at least one cardiac cycle, on one hand the diameter D(t) of the artery at a first location (23) and on the other hand the pressure p(t) of the arterial bed at a second location (25), said first and second locations being assumed to be separated by a distance Δx,

b) memorizing (3, 4, 5) at successive instants of the cardiac cycle pairs of values which include a diameter value D(t) and a pressure value p(t),

c) calculating (7, 6) on the basis of the thus memorized value pairs by a mathematical adjustment procedure the parameters α, β, γ, ... of a relationship of the diameter as a function of the pressure D(p) = D(p, α, β, γ, ...), such relationship being selected to take into account the behaviour of the artery,

d) calculating (8) on the basis of said parameters and of each pressure value p(t) initially measured, the propagation velocity c(p) of the pressure wave generated by the cardiac funtion,

e) calculating (8) for each value of propagation velocity c(p) thus established and taking into account said distance Δx (9) the course time Δt(p) = Δx/c(p) of the pressure wave between said first and second locations,

f) calculating (11) for each pressure value p(t) initially measured, a new pressure value p[t + Δt(p)] prevailing at said first location and

g) establishing (12), with the aid of the diameter value D(t) initially measured and said new pressure value at said first location, the pressure-diameter relationship D(p) of said artery at said first location.

**2.** Method according to claim 1, characteried by the fact that

h) steps c) to g) are repeated (13) until the dispersion of the value pairs obtained following step g) satisfy a predetermined criterion.

**3.** Method according to claim 2, characterized by the fact that step h) is effected in taking up again the values acquired following step g).

**4.** Method according to claim 2, characterized by the fact that step h) is effected in taking up again the vAlues acquired following step g) and in modifying the value of said distance Δx (14) assumed in proceeding with the calculation indicated during step e).

**5.** Method according to claim 1, characterized by the fact that

    i) steps e) to g) are repeated until the dispersion of the value pairs obtained following step g) satisfy a predetermined criterion in modifying (14) the value of said assumed distance Δx.

**6.** Method according to claim 1, characterized by the fact that, in order to calculate the propatation velocity c(p) of step d), the expression

$$c(p) = \sqrt{\frac{S}{\rho} \cdot \frac{dp}{dS}}$$

is used, where

$$S = \frac{\pi D^2}{4}$$

and where $\rho$ is the density of the blood.

**7.** Method according to claim 1, characterized by the fact that all or some of the results are visualized on a screen (29).

**8.** Device for carrying out the method according to claim 1, characterized by the fact that it includes two non-invasive sensors, the first being a diameter sensor (3) placed at a first location (23) and the second being a pressure sensor (4) placed at a second location (25) in the course of an artery (22), said first (23) and second (25) locations being supposed to be separated by a distance Δx, and in that it further comprises a calculator (28) for processing the values supplied by said sensors (3, 4) and a visualization screen (29).

**Patentansprüche**

**1.** Verfahren zum Etablieren des Verhältnisses Druck/Durchmesser einer Arterie (22) an einem gegebenen Punkt (23) ihres Verlaufs, **dadurch gekennzeichnet,** daß es die Aufeinanderfolge der nachstehenden Schritte umfaßt:

    a) man mißt in nicht invasiver Weise und gleichzeitig während mindestens eines kardialen Zyklus einerseits den Durchmesser D(t) der Arterie an einer ersten Stelle (23) und andererseits den Druck p(t) des arteriellen Bettes an einer zweiten Stelle (25), welche ersten und zweiten Stellen als durch eine Distanz Δx voneinander getrennt angenommen sind,

    b) man speichert (3, 4, 5) in aufeinanderfolgenden Zeitpunkten des kardialen Zyklus Wertepaare umfassend einen Wert des Durchmessers D(t) und einen Wert des Druckes p(t),

    c) man berechnet (7, 6) durch eine mathematische Justiermethode auf die abgespeicherten Wertepaare die Parameter α, β, γ, ... eines Verhältnisses des Durchmessers in Abhängigkeit von dem Druck D(p) = D(p, α, β, γ, ...), wobei das Verhältnis ausgewählt wird, um dem Verhalten der Arterie Rechnung zu tragen,

    d) man berechnet (8) ausgehend von diesen Parametern und jedem anfänglich gemessenen Druckwert p(t) die Ausbreitungsgeschwindigkeit c(p) der druch die kardiale Funktion erzeugten Druckwelle,

    e) man berechnet (10) für jeden Ausbreitungsgeschwindigkeitswert c(p), der so gewonnen worden ist und unter Berücksichtigung der Distanz Δx (9) die Laufzeit Δt(p) = Δx/c(p) der Druckwelle zwischen der ersten und der zweiten Stelle,

    f) man berechnet (11) für jeden anfänglich gemessenen Wert des Druckes p(t) einen neuen Wert des Druckes p[t + Δt(p)], der an der ersten Stelle herrscht, und

    g) man etabliert (12) mit Hilfe des anfänglich gemessenen Durchmesserwertes D(t) und des neuen Druckwertes an der ersten Stelle das Verhältnis Druck/Durchmesser D(p) der Arterie an der ersten Stelle.

**2.** Verfahren nach Anspruch 1, dadurch gekennzeichnet, daß

    h) man die Schritte c) bis g) wiederholt (13), bis die Dispersion der nach Schritt g) gewonnenen Wertepaare ein vorbestimmtes Kriterium erfüllt.

**3.** Verfahren nach Anspruch 2, dadurch gekennzeichnet, daß der Schritt h) ausgeführt wird unter Aufgreifen der Werte gewonnen infolge des Schrittes g).

**4.** Verfahren nach Anspruch 2, dadurch gekennzeichnet, daß der Schritt h) ausgeführt wird unter Aufgreifen der Werte, die infolge des Schrittes g) gewonnen worden sind und unter Modifizieren des Wertes der angenommenen Distanz Δx (14), wenn man zu der in Schritt e) vorgesehenen Berechnung fortfährt.

**5.** Verfahren nach Anspruch 1, dadurch gekennzeichnet, daß

i) man die Schritte e) bis g) wiederholt, bis die Dispersion der nach Schritt g) gewonnenen Wertepaare ein vorbestimmtes Kriterium erfüllt unter Modifizieren (14) des Wertes der angenommenen Distanz Δx.

6. Verfahren nach Anspruch 1, dadurch gekennzeichnet, daß für das Berechnen der Ausbreitungsgeschwindigkeit c(p) im Schritt d) die Gleichung verwendet wird

$$c(p) = \sqrt{\frac{S}{\rho} \cdot \frac{dp}{dS}}$$

worin

$$S = \frac{\pi D^2}{4}$$

und worin $\rho$ die Blutdichte sind.

7. Verfahren nach Anspruch 1, dadurch gekennzeichnet, daß man die Gesamtheit oder einen Teil der gewonnenen Resultate auf einem Bildschirm (29) visualisiert.

8. Vorrichtung für die Durchführung des Verfahrens nach Anspruch 1, dadurch gekennzeichnet, daß sie zwei nicht invasive Sensoren umfaßt, wobei der erste ein Sensor (3) für den Durchmesser ist, der an der ersten Stelle (23) plaziert wird, und der zweite ein Sensor (4) für den Druck ist, der an einer zweiten Stelle (25) des Verlaufs einer Arterie (22) plaziert wird, welche erste (23) und zweite (25) Stelle als durch eine Distanz Δx getrennt angenommen werden und daß sie ferner einen Rechner (28) für die Verarbeitung der Werte umfaßt, geliefert von den Sensoren (3, 4) sowie einen Visualisierungsbildschirm (29).

Fig.1

22
23
3
20
Δx
D(t)
29
28
4
25
26
21
ρ(t)

Fig. 2

Diamètre (mm)

4.45
4.40
4.35
4.30
4.25
4.20

D(t)

temps(s)

0    1    2    3

# Fig.3

# Fig.4

# Fig.5

Diamètre (mm)

$D(p) = D(p, \alpha, \beta, y, ...)$

Pression (mm Hg)

# Fig.6

Diamètre (mm)

Pression (mm Hg)

# Fig.7

# Fig.8

Fig.9

Compliance ( mm²/mmHg )

Pression (mm Hg)

Fig.10

Vitesse de l'onde (m/s)

Pression (mm Hg)

Fig.11

- Pression p(t) — 4
- 3 — Diamètre D(t)
- 5 Courbe pression-diamètre
- 7 Ajustement
- 6 relation $D(p)=D(p,\alpha,\beta,y,...)$
- 8 Calcul $c(p)$
- 9 estimation $\Delta x$
- 10 Calcul $\Delta t(p)$
- modification $\Delta x$ — 14
- 11 Correction $p(t) \to p(t+\Delta t)$
- 12 Courbe pression-diamètre corrigée
- 13 Hystérèse supprimée ?
  - non
  - non
  - oui
- 15 Ajustement final
- 16 Compliance, vitesse de propagation, etc...